Europäisches Patentamt

European Patent Office

Office européen des brevets

(19)

(11) Veröffentlichungsnummer: **0 261 513**
**A1**

(12) **EUROPÄISCHE PATENTANMELDUNG**

(21) Anmeldenummer: 87113287.4

(22) Anmeldetag: 11.09.87

(51) Int. Cl.⁴: **A61M 5/00** , A61M 5/31

(30) Priorität: **20.09.86 DE 3632092**

(43) Veröffentlichungstag der Anmeldung:
**30.03.88 Patentblatt 88/13**

(84) Benannte Vertragsstaaten:
**AT BE CH DE FR GB IT LI NL SE**

(71) Anmelder: **Bauer, Hans**
**Gutenbergerstrasse 20**
**D-7311 Dettingen(DE)**

(72) Erfinder: **Bauer, Hans**
**Gutenbergerstrasse 20**
**D-7311 Dettingen(DE)**

(74) Vertreter: **Cast, Adolf, Dipl.-Ing.**
**Schmaltalstrasse 13**
**D-7318 Lenningen 1(DE)**

(54) **Entsorgungssystem für Kanülen.**

(57) Bei einem Entsorgungssystem für Kanülen (2) für medizinische Spritzen (1) ist in einer Abdeckplatte (5) ein Formschlitz (6) vorgesehen, in den die Hülse (3) der Kanüle (2) einführbar ist. Damit lassen sich sowohl aufgesteckte als auch aufgeschraubte Kanülen (2) leicht von der Spritze (1) trennen, ohne daß der Benutzer mit der Kanüle (2) in Berührung kommt. Diese fällt vielmehr in einen Entsorgungsbehälter (8), der nach dem Füllen verschlossen wird und sicher entsorgt werden kann.

Eine Verletzung des Benutzers und eine Gefahr der Ansteckung durch gebrauchte Kanülen ist somit sicher ausgeschlossen.

FIG. 1

EP 0 261 513 A1

## Entsorgungssystem für Kanülen

Die Erfindung bezieht sich auf ein Entsorgungssystem für Kanülen bei medizinischen Spritzen gemäß Oberbegriff von Anspruch 1.

Bei solchen medizinischen Spritzen sind die Kanülen auf die Spritzen entweder aufgesteckt oder aufgeschraubt und es ist bekannt, diese Kanülen nach Gebrauch in einen Entsorgungsbehälter zu werfen. Dabei müssen sie von Hand abgezogen oder abgeschraubt werden, wobei immer die Gefahr einer Verletzung des Benützers vorhanden ist. Diese Gefahr ist besonders groß bei einer infektiösen Verunreinigung der Kanüle mit der damit verbundenen Ansteckungsgefahr.

Aufgabe der vorliegenden Erfindung ist daher die Schaffung eines Entsorgungssystems zur sicheren und bequemen Trennung der Kanüle von der Spritze, ohne daß der Benützer mit der Kanüle in Berührung kommt und wobei das Entsorgungssystem für alle Befestigungsarten universell verwendbar ist.

Diese Aufgabe wird erfindungsgemäß durch das im kennzeichnenden Teil des Anspruchs 1 angegebene Merkmal gelöst.

Der erfindungsgemäße Formschlitz erlaubt es, sowohl aufgesteckte als auch aufgeschraubte Kanülen von der Spritze zu trennen, ohne daß der Benützer mit der Kanüle in Berührung kommt. Diese gelangt vielmehr unmittelbar in den Entsorgungsbehälter und die Gefahr einer Ansteckung oder Verletzung des Benützers ist ausgeschlossen.

Durch die besondere Form des Formschlitzes ist dieser universell verwendbar, die aufgesteckte Kanüle lässt sich durch Ziehen oder Kippen von der Spritze lösen, ebenso ist es möglich, eine mit der Spritze verschraubte Kanüle von diesem bequem zu trennen.

Erfindungsgemäß hat der Formschlitz eine solche Länge, daß auch Infusionsschläuche mit angesetzten Lappen bequem in den Entsorgungsbehälter geworfen werden können.

Bei einer vorteilhaften Ausführungsform der Erfindung ist auf der Abdeckplatte ein Wulst vorgesehen, der zur Aufnahme eines Endes der Spritze dient. Beim Abschrauben dieser Spritze hebt sich diese dann so an, daß sie sich über einen Durchbruch im Wulst verschieben lässt. Dies ist gleichzeitig eine Anzeige, daß die Schraubverbindung zwischen Kanüle und Spritze gelöst ist und daß die Kanüle in den Entsorgungsbehälter abfallen kann.

Wenn die Abdeckplatte erfindungsgemäß mit einem Gestell verbunden ist, so lässt sich dieses bequem auf jede beliebige Fläche abstellen oder an der Wand befestigen. Damit eignet sich das Entsorgungssystem besonders für Privatpraxen und bietet eine einfache und billige Möglichkeit, gebrauchte Kanülen zu beseitigen.

Im folgenden sind Ausführungsbeispiele der Erfindung unter Bezugnahme auf die Zeichnungen näher beschrieben.

Es zeigen:

Fig. 1 Entsorgungssystem mit Gestell, Mittelschnitt

Fig. 2 Abdeckplatte mit Formschlitz, von oben

Fig. 3 Schnitt nach Linie III-III in Fig. 2

Fig. 4 Zweites Ausführungsbeispiel eines Entsorgungssystems, Mittelschnitt

Fig. 5 Weiteres Ausführungsbeispiel eines Formschlitzes, von oben

Fig. 6 Schnitt nach Linie VI-VI in Fig. 5

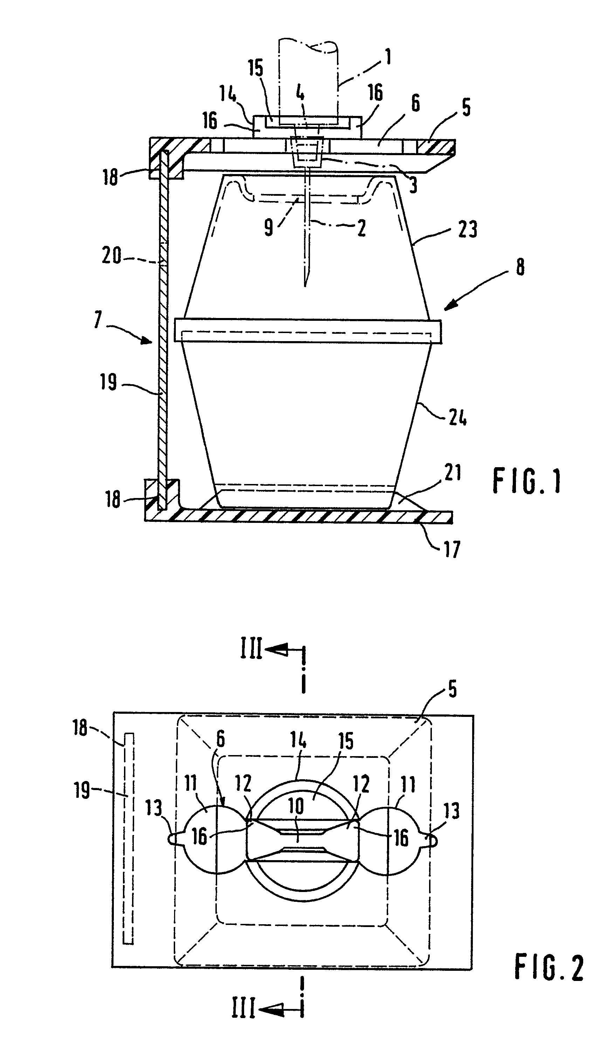

Fig. 1 zeigt das gesamte Entsorgungssystem, mit dem von einer Spritze 1 die Kanüle 2 getrennt werden soll. Diese ist über eine Hülse 3 mit einem Zapfen 4 mit der Spritze 1 verbunden. Im vorliegenden Fall handel es sich um ein Schraubgewinde.

Das Entsorgungssystem selbst besteht aus einer Abdeckplatte 5 mit durchgehendem Formschlitz 6 und einem Gestell 7 mit im wesentlichen U-förmigem Querschnitt, mit dem die Abdeckplatte 5 verbunden ist.

In das Gestell 7 lässt sich unter die Abdeckplatte 5 ein Entsorgungsbehälter 8 einschieben, der oben eine Öffnung 9 aufweist.

Wie Fig. 2 zeigt, weist der Formschlitz 6 einen Parallelschlitz 10 und erweiterte Durchbrüche 11 auf, wobei der Parallelschlitz 10 mit einem konischen Zwischenschlitz 12 in den Durchbruch 11 übergeht. In Verlängerung der Durchbrüche 11 sind Kerben 13 vorgesehen.

Im Bereich von Parallelschlitz 10 ist mit der Abdeckplatte 5 ein kreisförmiger Wulst 14 verbunden, der eine Vertiefung 15 und zwei seitliche Schlitze 16 aufweist, die mit dem Parallelschlitz fluchten.

Das Gestell 7 weist eine Bodenplatte 17 auf, die mit einer Nut 18 versehen ist. In diese Nut 18 ist eine Rückenplatte 19 eingesteckt, an deren freiem Ende die Abdeckplatte 5 ebenfalls mit einer Nut 18 eingesteckt ist. Diese Rückenplatte 19 kann verschiedene Längen aufweisen, so daß verschieden hohe Entsorgungsbehälter 8 in das Gestell 7 eingeführt werden können.

Die Rückenplatte 19 weist ferner Durchbrüche 20 auf, mit denen das Gastell an einer Wand aufhängbar ist.

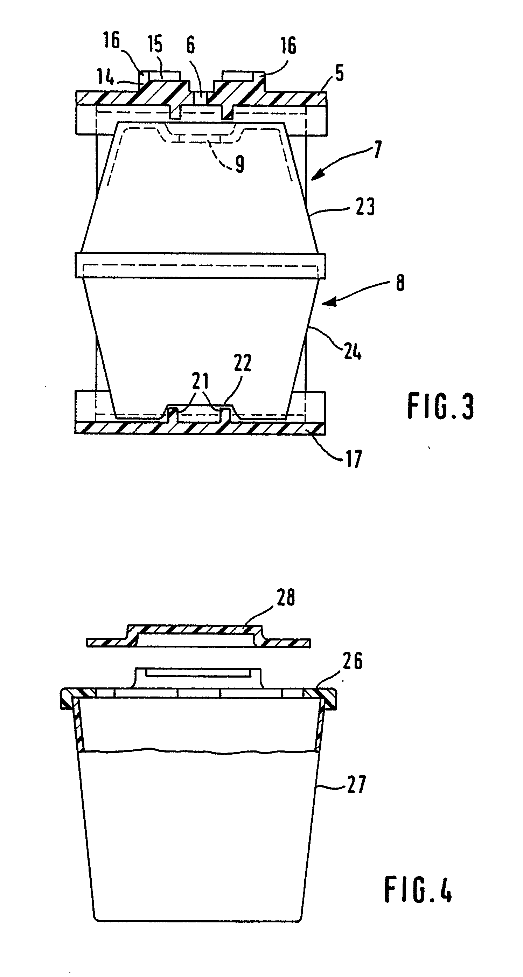

Die Bodenplatte 17 ist mit zwei Rippen 21 versehen, die in eine Rinne 22 von Entsorgungsbehälter 8 eingreifen(Fig 3). Damit ist gewährleistet, daß nur zulässige Entsorgungsbehälter 8 verwendet werden, die gewährleisten, daß deren Wände nicht von den entsorgten Kanülen durchstoßen werden.

Der Entsorgungsbehälter 8 selbst besteht aus zwei ineinander steckbaren Schalen 23 und 24. Dabei weist die obere Schale 23 eine Offnung 9 auf, durch den die Kanüle 2 in den Entsorgungsbehälter 8 hineinfällt und der nach der Entnahme aus dem Gestell 7 durch einen nicht dargestellten Deckel verschließbar ist.

Bei einem weiteren Ausführungsbeispiel gemäß Fig. 4 ist die Abdeckplatte 26 direkt auf einen Entsorgungsbehälter 27 aufgesteckt. Ist dieser mit Kanülen gefüllt, dann wird er durch einen Deckel 28 verschlossen und kann so gefahrlos entsorgt werden.

Bei der Handhabung des erfindungsgemäßen Entsorgungssystems, wobei eine aufgesteckte Kanüle 2 von der Spritze 1 durch Abhebeln getrennt werden soll, wird die Hülse 3 so in den Durchbruch 11 gesteckt, daß die Kanüle 2 in den Entsorgungsbehälter 8 ragt, während ihre Hülse 3 in den Zwischenschlitz 12 geschoben wird. Wird jetzt die Spritze 1 durch Ziehen oder Kippen nach oben abgehoben, dann fällt die Kanüle 2 in den Entsorgungsbehälter 8.

Bei verschraubten Kanülen 2 weist deren Hülse 3 einen Vierkant auf, der in den Parallelschlitz 10 passt. Das Ende der Spritze 1 wird in die Vertiefung 15 von Wulst 14 gesteckt, die Kanüle 2 ragt wieder in den Entsorgungsbehälter 8. Beim Drehen der Spritze 1 wird die Hülse 3 vom Parallelschlitz 10 festgehalten, durch die Schraubbewegung verschiebt sich die Spritze 1 nach oben, bis die Kanüle 2 abfällt. Dann lässt sich die Spritze 1 durch einen der Schlitze 16 nach außen verschieben und abnehmen.

Die Kerben 13 dienen dazu, daß der Zapfen 4 der Spritze 1 abgebrochen oder daß die Kanüle 2 verbogen werden kann. Damit wird die ganze Spritze unbrauchbar und kann von Unbefugten nicht mehr verwendet werden.

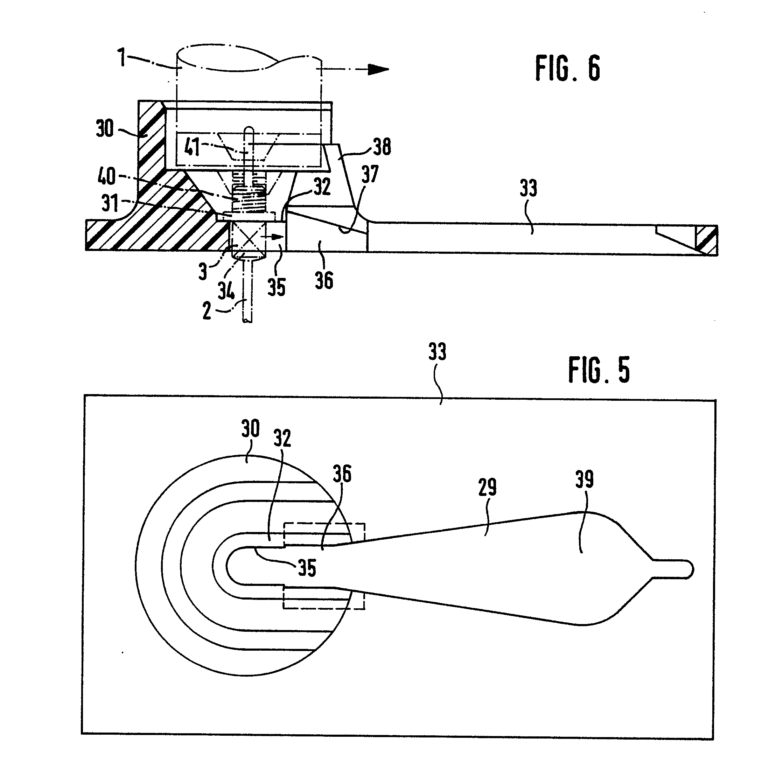

Bei einem weiteren Ausführungsbeispiel des Formschlitzes 29 gemäß Fig. 5 und 6 ist der Wulst 30 an einem Ende des Formschlitzes angeordnet. Er umfasst damit die Spritze 1 über einen möglichst großen Umfangsbereich und gewährleistet eine sichere Führung dieser Spritze.

Wie Fig. 6 zeigt, handelt es sich bei diesem Ausführungsbeispiel darum, eine mit der Spritze 1 verschraubte Kanüle 2 von dieser sicher zu trennen. Dabei weist die Hülse 3 der Kanüle 2 einen Bund 31 auf, der bei eingeführter Spritze 1 an einer Auflagefläche 32 der Abdeckplatte 33 anliegt. Ein Vierkant 34 der Hülse 3 passt in den Parallelschlitz 35 von Formschlitz 29.

Im Anschluß an den Parallelschlitz 35 weist der Formschlitz 29 einen Abstreifschlitz 36 auf, in dessen Bereich zu beiden Seiten in der Abdeckplatte 33 nach unten geneigte Abstreifflächen 37 vorgesehen sind.

Zum Trennen der Kanüle 2 von der Spritze 1 wird diese um 360° verdreht und hebt sich soweit an, daß sie sich durch einen Schlitz 38 im Wulst 30 in Richtung Durchbruch 39 verschieben lässt. Das Gewinde 40 zwischen Spritze 1 und Hülse 3 ist frei, Hülse 3 wird jedoch über eine Verlängerung 41 mitgenommen, so daß sich der Bund 31 unter die Abstreiffläche 37 schiebt, wobei Hülse 3 sicher und zwangsläufig von der Spritze 1 getrennt wird und in den Entsorgungsbehälter 8 abfällt>

## Ansprüche

1) Entsorgungssystem für Kanülen bei medizinischen Spritzen, wobei die Kanüle eine Hülse aufweist, mit der sie mit einem Zapfen der Spritze abnehmbar verbunden ist und mit einem Entsorgungsbehälter zur Aufnahme der gebrauchten Kanülen, **dadurch gekennzeichnet,** daß in einer Abdeckplatte (5,26) ein durchgehender Formschlitz (6) vorgesehen ist, in den die Hülse (3) zum Trennen der Kanüle (2) von der Spritze (1) einführbar ist.

2) Entsorgungssystem nach Anspruch 1, **dadurch gekennzeichnet,** daß der Formschlitz (6) einen Parallelschlitz (10) aufweist, der in mindestens einen erweiterten Durchbruch (11) mündet.

3) Entsorgungssystem nach Anspruch 2, **dadurch gekennzeichnet,** daß zwischen Parallelschlitz (10) und Durchbruch (11) ein konischer Zwischenschlitz (12) vorgesehen ist.

4) Entsorgungssystem nach einem der Ansprüche 1 bis 3, **dadurch gekennzeichnet,** daß der Formschlitz (6) mindestens eine Kerbe (13) aufweist.

5) Entsorgungssystem nach einem der Ansprüche 1 bis 4, **dadurch gekennzeichnet,** daß die Abdeckplatte (5,26) im Bereich des Parallelschlitzes (10) einen Wulst (14) zur Aufnahme eines Endes der Spritze (1) aufweist.

6) Entsorgungssystem nach Anspruch 5, **dadurch gekennzeichnet,** daß der Wulst (14) mindestens einen Schlitz (16) aufweist, der mit dem Parallelschlitz (10) fluchtet.

7) Entsorgungssystem nach Anspruch 6, **dadurch gekennzeichnet**, daß zwischen Parallelschlitz (35) und Durchbruch (39) ein Abstreifelement (37) zum zwangsläufigen Abstreifen der Kanüle (2) vorgesehen ist.

8) Entsorgungssystem nach Anspruch 7, **dadurch gekennzeichnet**, daß das Abstreifelement (37) als geneigte Abstreiffläche ausgebildet ist.

9) Entsorgungssystem nach einem der Ansprüche 1 bis 8, **dadurch gekennzeichnet**, daß die Abdeckplatte (5,33) mit einem Gestell (7) verbunden ist, in das der Entsorgungsbehälter (8) einschiebbar ist.

10) Entsorgungssystem nach Anspruch 9, **dadurch gekennzeichnet**, daß eine Rückenplatte (19) leicht lösbar mit der Abdeckplatte (5,33) und der Bodenplatte (17) verbunden ist.

11) Entsorgungssystem nach Anspruch 9 oder 10, **dadurch gekennzeichnet**, daß das Gestell (7) einen im wesentlichen U-förmigen Querschnitt aufweist und an seiner Rückenplatte (19) mit Durchbrüchen (20) zur Befestigung an einer Wand vorgesehen ist.

12) Entsorgungssystem nach einem der Ansprüche 1 bis 11, **dadurch gekennzeichnet**, daß der Entsorgungsbehälter (8) zwei sich ergänzende Schalen (23,24) aufweist, die miteinander verbindbar sind.

13) Entsorgungssystem nach Anspruch 12, **dadurch gegekennzeichnet**, daß die untere Schale (24) eine Rinne (22) aufweist, in die Rippen (21) der Bodenplatte (17) eingreifen.

14) Entsorgungssystem nach einem der Ansprüche 1 bis 8, **dadurch gekennzeichnet**, daß die Abdeckplatte (26) mit einem Entsorgungsbehälter (27) verbunden ist.

FIG.1

FIG.2

FIG.3

FIG.4

FIG. 6

FIG. 5

Europäisches
Patentamt

# EUROPÄISCHER RECHERCHENBERICHT

## EINSCHLÄGIGE DOKUMENTE

EP 87113287.4

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP - A1 - 0 136 392 (HSIEH CH.) | 1,2 | A 61 M 5/00 |
| Y | * Gesamt; insbesondere Fig. 2,5,6; Seite 3, Absatz 1; Seite 4, Absätze 3,4 * | 9,14 | A 61 M 5/31 |
| A | | 7,8 | |
| | -- | | |
| Y | DE - A1 - 2 948 632 (G.A.B.AUSTIN) | 9 | |
| A | * Fig. 1; Seite 7, Absatz 3 * | 11 | |
| | -- | | |
| Y | DE - A - 2 161 830 (LANDSTINGENS) | 14 | |
| | * Fig. 1; Seite 2, Absatz 7 * | | |
| | ---- | | |

| RECHERCHIERTE SACHGEBIETE (Int. Cl.4) |
|---|
| A 61 M 5/00 |

Der vorliegende Recherchenbericht wurde für alle Patentansprüche erstellt.

| Recherchenort | Abschlußdatum der Recherche | Prüfer |
|---|---|---|
| WIEN | 15-01-1988 | LUDWIG |